# EUROPEAN PATENT APPLICATION

(11) **EP 3 750 922 A1**
(43) Date of publication of application: **16.12.2020**
(21) Application number: 19747376.2
(22) Date of filing: 02.02.2019
(51) Int. Cl.: C08B 37/00, A61K 31/715, A61P 3/06

(54) **SEPARATED IMPERATA CYLINDRICA POLYSACCHARIDE AND USE THEREOF**

(30) Priority: 05.02.2018 CN 201810114169
(71) Applicant: Shanghai Green Valley Pharmaceutical Co., Ltd., Pudong New Area, Shanghai 201203 (CN); Soochow University, Suzhou, Jiangsu 215213 (CN)
(72) Inventor: ZHANG, Zhenqing, Shanghai 201203 (CN); XU, Naiyu, Suzhou, Jiangsu 215123 (CN); YIN, Xiang, Shanghai 201203 (CN); PANG, Li, Shanghai 201203 (CN); SHEN, Lulu, Shanghai 201203 (CN); XUE, Jie, Suzhou, Jiangsu 215123 (CN)
(74) Representative: Plasseraud IP
(86) International application number: PCT/CN2019/074616
(87) International publication number: WO 2019/149285

(57) **Abstract**

Provided herein are an isolated *Imperata cylindrica* polysaccharide and use thereof in the manufacture of a medicament for treating hyperlipoidemia. The *Imperata cylindrica* polysaccharide comprises *L*-arabinose, *D*-xylose, *D*-mannose, *D*-glucose and *D*-galactose, wherein the molar ratio of the *L*-arabinose: *D*-xylose: *D*-mannose: *D*-glucose: *D*-galactose is 1-20 : 1-15 : 1-15 : 15-40 : 25-60.

## Description

### TECHNICAL FIELD

The present application relates to the field of medicine. Specifically, the present application relates to an isolated *Imperata cylindrica* polysaccharide (ICP) and use thereof in the manufacture of a medicament for treating hyperlipoidemia.

### BACKGROUND OF THE INVENTION

Researches in recent years have found that carbohydrates are not only a type of important structural and energy substances, but also have important biological functions. Carbohydrates are involved in the processes of mutual recognition and information transmission between cells, and are considered as another type of important informational molecules besides nucleic acids in organisms. Moreover, carbohydrates are also key factors for cell surface signal recognition, antigen-antibody reactions, and information transmission and perception between cells. Therefore, researches on polysaccharides with biological activities attract increasing attention. Due to the complex structures of carbohydrates, their separation and structural identification are difficult. So far, only coriolus versicolor polysaccharide, polyporus polysaccharide, lentinan, schizophyllan, pachymaran and the like have been used clinically. There is a need for more bioactive polysaccharides in the art.

The traditional Chinese medicinal material *Imperata cylindrica* refers to is the dried rhizome of *Imperata cylindrica* (Linn) Beauv. var. major (Nees) C. E. Hubb., an *Umbelliferae* plant. The main chemical components of *Imperata cylindrica* include carbohydrates, triterpenes, lactones, organic acids, etc. *Imperata cylindrica* polysaccharide is a branched polysaccharide formed by connection of multiple monosaccharides. Generally, *Imperata cylindrica* polysaccharides are characterized by the composition of the monosaccharides contained therein and the mode of connection thereof. The *Imperata cylindrica* polysaccharides prepared by different extraction methods have different monosaccharide compositions and connection modes. Yike Zou, *et al.* (Yike Zou, Mingyue Zhang, Caiyun Wang, et al., Isolation of Imperata cylindrica polysaccharide IC1 and determination of its relative molecular weight and monosaccharide composition, Chinese Journal of Experimental Traditional Medical Formulae, 2012, 18(2): 80-82) reported an *Imperata cylindrica* polysaccharide having a molecular weight of 8292.2, comprising rhamnose, xylose, fructose, mannose, and glucose at a molar ratio of 1 : 11.45 : 1.26 : 1.02 : 95.23. *Imperata cylindrica* polysaccharides are generally used in the art for hemostasis, immunoregulation, diuresis and blood pressure reduction, antibacterial, anti-inflammation and analgesic, anti-tumor, anti-oxidation, renal function improvement, etc. There is no report about use of *Imperata cylindrica* polysaccharide for treatment of hyperlipoidemia till now.

Hyperlipoidemia refers to a metabolic disease in which the levels of one or more lipids in the blood are abnormal (for example, multiple lipids are present in a level higher than the normal level). Hyperlipoidemia is manifested as too high levels of total cholesterol (TC), triglyceride (TG) and low-density lipoprotein cholesterol (LDL-C) or a too low level of high-density lipoprotein cholesterol (HDL-C) in the blood. In recent years, the incidence of hyperlipoidemia increasingly grows. Hyperlipoidemia is also closely associated with some severe cardiovascular and cerebrovascular diseases such as atherosclerosis and coronary heart disease.

### SUMMARY OF THE INVENTION

Due to the complex structures of carbohydrates, different extraction processes will directly affect the structural composition of polysaccharides, thereby affecting their efficacies. The present invention provides an improved method for preparing *Imperata cylindrica* polysaccharide, comprising a step of gradient precipitation. It was found from structural identification that the isolated *Imperata cylindrica* polysaccharide of the present invention had a completely different structure from the known *Imperata cylindrica* polysaccharides. It was verified by animal experiments that the isolated *Imperata cylindrica* polysaccharide of the present invention had a potential effect of regulating blood lipids.

In one aspect, the present application provides an isolated *Imperata cylindrica* polysaccharide, comprising monosaccharides such as *L*-arabinose (*L*-Ara), *D*-xylose (*D*-Xyl), *D*-mannose (*D*-Man), *D*-glucose (*D*-Glc) and *D*-galactose (*D*-Gal), wherein the molar ratio of the *L*-arabinose: *D*-xylose: *D*-mannose: *D*-glucose: *D*-galactose is 1-20 : 1-15 : 1-15 : 15-40 : 25-60, preferably 5-10 : 1-5 : 1-5 : 25-30 : 45-55.

In one embodiment, the monosaccharide components contained in the isolated *Imperata cylindrica* polysaccharide are connected to each other through glycosidic bonds. The *L*-arabinose includes terminal *L*-arabinose and/or 1,2,4-linked *L*-arabinose; the *D*-xylose includes terminal *D*-xylose and/or 1,3,4-linked *D*-xylose; the *D*-mannose includes terminal *D*-mannose and/or 1,6-linked *D-*mannose; the *D*-glucose includes 1,4-linked *D*-glucose and/or 1,6-linked *D*-glucose; or the *D-*galactose includes 1,4-linked *D*-galactose and/or 1,4,6-linked *D*-galactose.

In one preferred embodiment, the *L*-arabinose as described in the present application includes terminal *L*-arabinose and/or 1,2,4-linked *L*-arabinose.

In one preferred embodiment, the *D*-xylose as described in the present application includes terminal *D*-xylose and/or 1,3,4-linked *D*-xylose.

In one preferred embodiment, the *D*-mannose as described in the present application includes terminal *D*-mannose and/or 1,6-linked *D*-mannose.

In one preferred embodiment, the *D*-glucose as described in the present application includes 1,4-linked *D*-glucose and/or 1,6-linked *D*-glucose.

In one preferred embodiment, the *D*-galactose as described in the present application includes 1,4-linked *D*-galactose and/or 1,4,6-linked *D*-galactose.

In one embodiment, the isolated *Imperata cylindrica* polysaccharide comprises terminal *L*-arabinose, 1,2,4-linked *L*-arabinose, terminal *D*-xylose, 1,3,4-linked *D*-xylose, terminal *D*-mannose, 1,6-linked *D*-mannose, 1,4-linked *D*-glucose, 1,6-linked *D*-glucose, 1,4-linked *D*-galactose, and 1,4,6-linked *D-*galactose. In another preferred embodiment, the molar ratio of the terminal *L*-arabinose: 1,2,4-linked *L*-arabinose: terminal *D*-xylose: 1,3,4-linked *D*-xylose: terminal *D*-mannose: 1,6-linked *D*-mannose: 1,4-linked *D*-glucose: 1,6-linked *D*-glucose: 1,4-linked *D*-galactose: 1,4,6-linked *D*-galactose is 1-10 : 1-10 : 1-5 : 1-10 : 1-5 : 1-10 : 15-30 : 1-10 : 10-25 : 15-30, preferably 1-5 : 1-5 : 1-3 : 1-5 : 1-3 : 1-5 : 20-25 : 1-5 : 15-20 : 20-25.

In another embodiment, one or more of the monosaccharide components are pyranose. In one preferred embodiment, the monosaccharide components are all pyranose.

In one preferred embodiment, the isolated *Imperata cylindrica* polysaccharide as described in the present application has a molecular weight ranging from 1×10⁴ to 5×10⁵ Da, preferably 1×10⁵ to 3×10⁵ Da.

In another aspect, the present application provides a method for preparing the isolated *Imperata cylindrica* polysaccharide, comprises the steps of:
(1) extracting *Imperata cylindrica* with water one or more times to give an aqueous extract of *Imperata cylindrica,* optionally concentrating the aqueous extract of *Imperata cylindrica;*
(2) adding an organic solvent to the optionally concentrated aqueous extract of *Imperata cylindrica* to give an mixture containing the organic solvent at a concentration of 15-30%, preferably 17-28%, and more preferably 20-25%, centrifuging the mixture to give a supernatant;
(3) adding an organic solvent to the supernatant to give a mixture containing the organic solvent at a concentration of 70-90%, preferably 75-85%, and more preferably 80-85%, centrifuging the mixture to give a precipitate; and
(4) drying the precipitate to obtain the isolated *Imperata cylindrica* polysaccharide.

In one embodiment, the concentration of the organic solvent in step (2) is preferably 17-28%, more preferably 20-25%. In some embodiments, step (2) is also referred to as the first gradient precipitation.

In one embodiment, the concentration of the organic solvent in step (3) is preferably 75-85%, more preferably 80-85%.

In one embodiment, the volume : weight ratio of water to the *Imperata cylindrica* in step (1) is 8:1 to 30:1, preferably 20:1 to 30:1.

In one embodiment, the extraction temperature in step (1) is 40-100 °C, preferably 60-100 °C, more preferably 80-100 °C, and most preferably 90-95 °C.

In one embodiment, the extraction time in step (1) is 1-4 hours, preferably 1-2 hours.

In one embodiment, in step (1), the *Imperata cylindrica* is extracted with water 1, 2, 3, or 4 times.

In one embodiment, the method further comprises step (3') between steps (3) and (4): dissolving the precipitate obtained from step (3) with water to give an aqueous solution, adding an organic solvent to the aqueous solution to give a mixture containing the organic solvent at a concentration of 70-90%, preferably 75-85%, and more preferably 80-85%, and centrifuging the mixture to give a further precipitate; wherein step (3') can be repeated one or more times, preferably 1, 2 or 3 times.

In some embodiments, steps (3) and/or (3') are also referred to as the second gradient precipitation.

In one embodiment, the organic solvent in step (2) and/or (3) and/or (3') is selected from methanol, ethanol, propanol, acetone, or a mixture thereof, preferably ethanol.

The *Imperata cylindrica* as described in the present application include the commercially available medicinal material *Imperata cylindrica* (i.e., dried rhizome of the plant *Imperata cylindrica*) and decoction pieces of *Imperata cylindrica.* In one embodiment, the *Imperata cylindrica* as described in the present application is a decoction piece of *Imperata cylindrica.*

The term "isolated *Imperata cylindrica* polysaccharide" refers to the *Imperata cylindrica* polysaccharide isolated from the natural state where its original plant raw material exists naturally by an artificial means (such as extraction, purification, or the like). The plant raw material can be *Imperata cylindrica* in the form of a plant or *Imperata cylindrical* in the form of a medicinal material, such as dried rhizome of the plant *Imperata cylindrical* or decoction pieces of *Imperata cylindrica.*

In one embodiment, the present application provides an isolated *Imperata cylindrica* polysaccharide, which is obtained by the method of preparation as described in the present application. In one preferred embodiment, the isolated *Imperata cylindrica* polysaccharide comprises *L*-arabinose, *D-*xylose, *D*-mannose, *D*-glucose and *D*-galactose, wherein the molar ratio of the *L*-arabinose: *D-*xylose: *D*-mannose: *D*-glucose: *D*-galactose is 1-20 : 1-15 : 1-15 : 15-40 : 25-60, preferably 5-10 : 1-5 : 1-5 : 25-30 : 45-55. In one preferred embodiment, the *L*-arabinose includes terminal *L*-arabinose and/or 1,2,4-linked *L*-arabinose; the *D*-xylose includes terminal *D*-xylose and/or 1,3,4-linked *D-*xylose; the *D*-mannose includes terminal *D*-mannose and/or 1,6-linked *D*-mannose; the *D*-glucose includes 1,4-linked *D*-glucose and/or 1,6-linked *D*-glucose; and the *D*-galactose includes 1,4-linked *D*-galactose and/or 1,4,6-linked *D*-galactose. In a further preferred embodiment, the molar ratio of the terminal *L*-arabinose: 1,2,4-linked *L*-arabinose: terminal *D*-xylose: 1,3,4-linked *D*-xylose: terminal *D*-mannose: 1,6-linked *D*-mannose: 1,4-linked *D*-glucose: 1,6-linked *D*-glucose: 1,4-linked *D*-galactose: 1,4,6-linked *D*-galactose is 1-10 : 1-10 : 1-5 : 1-10 : 1-5 : 1-10 : 15-30 : 1-10 : 10-25 : 15-30, preferably 1-5 : 1-5 : 1-3 : 1-5 : 1-3 : 1-5 : 20-25 : 1-5 : 15-20 : 20-25.

The terminal *L*-arabinose refers to *L*-arabinose connected to an adjacent group (e.g., an adjacent monosaccharide residue) through a glycosidic bond at position 1 of the sugar ring.

The 1,2,4-linked *L*-arabinose refers to *L*-arabinose connected to adjacent groups (e.g., adjacent monosaccharide residues) through glycosidic bonds at positions 1, 2 and 4 of the sugar ring.

The terminal *D*-xylose refers to *D*-xylose connected to an adjacent group (e.g., an adjacent monosaccharide residue) through a glycosidic bond at position 1 of the sugar ring.

The 1,3,4-linked *D*-xylose refers to *D*-xylose connected to adjacent groups (e.g., adjacent monosaccharide residues) through glycosidic bonds at positions 1, 3 and 4 of the sugar ring.

The terminal *D*-mannose refers to *D*-mannose connected to an adjacent group (e.g., an adjacent monosaccharide residue) through a glycosidic bond at position 1 of the sugar ring.

The 1,6-linked *D*-mannose refers to *D*-mannose connected to adjacent groups (e.g., adjacent monosaccharide residues) through glycosidic bonds at positions 1 and 6 of the sugar ring.

The 1,4-linked *D*-glucose refers to *D*-glucose connected to adjacent groups (e.g., adjacent monosaccharide residues) through glycosidic bonds at positions 1 and 4 of the sugar ring.

The 1,6-linked *D*-glucose refers to *D*-glucose connected to adjacent groups (e.g., adjacent monosaccharide residues) through glycosidic bonds at positions 1 and 6 of the sugar ring.

The 1,4-linked *D*-galactose refers to *D*-galactose connected to adjacent groups (e.g., adjacent monosaccharide residues) through glycosidic bonds at positions 1 and 4 of the sugar ring.

The 1,4,6-linked *D*-galactose refers to *D*-galactose connected to adjacent groups (e.g., adjacent monosaccharide residues) through glycosidic bonds at positions 1, 4 and 6 of the sugar ring.

The sugar as described in the present application can be in α-configuration or β-configuration.

In another aspect, the present application provides use of the isolated *Imperata cylindrica* polysaccharide obtained in the present invention in the manufacture of a medicament for treating hyperlipoidemia.

In one embodiment, the medicament for treating hyperlipoidemia is a blood lipid metabolism regulator.

In another aspect, the present application provides a pharmaceutical composition, comprising a therapeutically effective amount of the isolated *Imperata cylindrica* polysaccharide obtained in the present invention, and a pharmaceutically acceptable carrier.

In one preferred embodiment, the pharmaceutical composition is a tablet, capsule, granule, syrup, suspension, solution, dispersion, sustained-release formulation for oral or non-oral administration, formulation for intravenous injection, formulation for subcutaneous injection, inhalation formulation, transdermal formulation, rectal or vaginal suppository.

The pharmaceutically acceptable carrier as described in the present application refers to a pharmaceutically acceptable carrier well known to those skilled in the art. The pharmaceutically acceptable carriers in the present application include, but are not limited to, fillers, wetting agents, binders, disintegrants, lubricants, adhesives, glidants, taste-masking agents, surfactants, preservatives, etc. Fillers include, but are not limited to, lactose, microcrystalline cellulose, starch, powdered sugar, dextrin, mannitol, calcium sulfate, etc. Wetting agents and binders include, but are not limited to, sodium carboxymethyl cellulose, hydroxypropyl cellulose, hydroxypropyl methyl cellulose, gelatin, sucrose, polyvinylpyrrolidone, etc. Disintegrants include, but are not limited to, sodium starch glycolate, cross-linked polyvinylpyrrolidone, cross-linked sodium carboxymethyl cellulose, low-substituted hydroxypropyl cellulose, etc. Lubricants include, but are not limited to, magnesium stearate, micronized silica gel, talc, hydrogenated vegetable oil, polyethylene glycol, magnesium lauryl sulfate, etc. Adhesives include, but are not limited to, arabic gum, alginic acid, calcium carboxymethyl cellulose, sodium carboxymethyl cellulose, dextrates, dextrin, dextrose, ethyl cellulose, gelatin, liquid glucose, guar gum, hydroxyethyl cellulose, hydroxypropyl cellulose, hydroxypropyl methyl cellulose, magnesium aluminum silicate, maltodextrin, methyl cellulose, polymethacrylate, polyvinylpyrrolidone, pre-gelatinized starch, sodium alginate, sorbitol, starch, syrup and tragacanth. Glidants include, but are not limited to, colloidal silicon dioxide, powdered cellulose, magnesium trisilicate, silicon dioxide, and talc. Taste-masking agents include, but are not limited to, aspartame, stevioside, fructose, glucose, syrup, honey, xylitol, mannitol, lactose, sorbitol, maltitol, and glycyrrhizin. Surfactants include, but are not limited to, Tween-80, and poloxamer. Preservatives include, but are not limited to, paraben, sodium benzoate, potassium sorbate, etc.

Methods for preparing various pharmaceutical compositions comprising active ingredients in various ratios are known in the art or are obvious to those skilled in the art according to the disclosure of the present application, as described in REMINGTON'S PHARMACEUTICAL SCIENCES, Martin, E.W., ed., Mack Publishing Company, 19th ed. (1995). The method for preparing the pharmaceutical composition comprises incorporating a suitable pharmaceutical excipient, carrier, diluent, or the like. The pharmaceutical composition described in the present application is prepared by a known method, including conventional mixing, dissolving or freeze-drying processes.

In the pharmaceutical composition described in the present application, the proportion of the active ingredient can vary from about 0.01% to about 99% of the weight of a given unit dosage form. In such therapeutically useful pharmaceutical composition formulations, the amount of the active ingredient is such that an effective dosage level can be achieved.

The tablet, capsule or the like as described in the present application can comprise: a binder, such as tragacanth, arabic gum, corn starch, or gelatin; an excipient, such as dicalcium hydrogen phosphate; a disintegrant, such as corn starch, potato starch, alginic acid, or the like; a lubricant, such as magnesium stearate; a sweetener, such as sucrose, fructose, lactose, or aspartame; or a flavoring agent, such as peppermint, wintergreen oil, or cherry flavor. When the unit dosage form is a capsule, in addition to the above types of materials, it can comprise a liquid carrier such as vegetable oil or polyethylene glycol. Various additional materials may be present as a coating or otherwise modify the physical form of the solid unit dosage form. For example, the tablet or capsule can be coated with gelatin, wax, shellac, sugar, or the like. The syrup can comprise an active ingredient, sucrose or fructose as a sweetener, methyl or propyl paraben as a preservative, a dye and a flavoring agent (such as cherry flavor or orange flavor). Of course, any material used for preparing any unit dosage form should be pharmaceutically acceptable and non-toxic in the amount used. In addition, the active ingredient can be incorporated into a sustained-release formulation and a sustained-release device.

The active ingredient can also be administered intravenously or intraperitoneally by infusion or injection. A solution of the active ingredient or a salt thereof in water can be prepared, and optionally mixed with a non-toxic surfactant. A dispersion in glycerin, liquid polyethylene glycol, glyceryl triacetate, or a mixture thereof, or an oil can also be prepared. Such formulations comprise a preservative to prevent the growth of microorganisms under ordinary conditions of storage and use.

The dosage form of the pharmaceutical composition suitable for injection or infusion can include a sterile aqueous solution, dispersion or sterile powder comprising an active ingredient (optionally, encapsulated in a liposome) suitable for an immediate formulation of a sterile injectable or infusible solution or dispersion. In all cases, the final dosage form must be sterile, liquid and stable under the conditions of production and storage. The liquid carrier can be a solvent or a liquid dispersion medium, including, for example, water, ethanol, polyol (such as, glycerol, propylene glycol, liquid polyethylene glycol, or the like), vegetable oil, non-toxic glyceride, or a suitable mixture thereof. The proper fluidity can be maintained, for example, by formation of liposomes, by maintaining the desired particle size in the case of a dispersion, or by use of a surfactant. Various antibacterial and antifungal agents (such as parabens, chlorobutanol, phenol, sorbic acid, thimerosal, etc.) can be used to prevent microorganisms. In many cases, it is preferable to include an isotonic agent such as a sugar, buffer or sodium chloride. Prolonged absorption of an injectable composition can be produced by using a composition that delays absorption (for example, aluminum monostearate and gelatin).

A sterile injectable solution is prepared by combining the required amount of the active ingredient in a suitable solvent with various additional ingredients listed above as required, followed by sterile filtration. In the case of sterile powders for preparing a sterile solution for injection, preferred methods of preparation are vacuum drying and freeze-drying techniques, which produce a powder of the active ingredient plus any other required components present in the sterile filtered solution.

Useful solid carriers include pulverized solids (such as talc, clay, microcrystalline cellulose, silica, alumina, etc.). Useful liquid carriers include water, ethanol or ethylene glycol, or a water-ethanol/ethylene glycol mixture, in which the pharmaceutical composition of the present application can be dissolved or dispersed in an effective amount optionally with the help of a non-toxic surfactant. An adjuvant (such as fragrance) and an additional antimicrobial agent can be added to optimize the property for a given use.

A thickener (such as a synthetic polymer, fatty acid, fatty acid salt and ester, fatty alcohol, modified cellulose or modified inorganic material) can also be used with a liquid carrier to form a coatable paste, gel, ointment, soap, or the like, which can be directly applied to the user's skin.

The therapeutically effective amount of the active ingredient not only depends on the specific salt selected, but also depends on the mode of administration, the nature of the disease to be treated, and the age and status of the patient, and ultimately depends on the decision of the attending physician or clinician.

The above formulations can be presented in a unit dosage form, which is physically discrete units containing a unit dose, and is suitable for administration to human and other mammals. The unit dosage form can be a capsule or a tablet. Depending on the specific treatment involved, the unit dose of the active ingredient can vary from or be adjusted between about 0.01 to about 1000 mg or more. In another aspect, the present application provides use of a pharmaceutical composition comprising a therapeutically effective amount of the isolated *Imperata cylindrica* polysaccharide obtained by the present invention in the manufacture of a medicament for treating hyperlipoidemia.

In one embodiment, the medicament for treating hyperlipoidemia is a blood lipid metabolism regulator.

In another aspect, the present application provides an isolated *Imperata cylindrica* polysaccharide, which is obtained according to the method described in the present application.

In yet another aspect, the present application provides a method for treating hyperlipoidemia, comprising administering to a subject in need thereof a therapeutically effective amount of the isolated *Imperata cylindrica* polysaccharide obtained by the present invention. In one embodiment, the treatment of hyperlipoidemia refers to regulation of blood lipid metabolism.

In one preferred embodiment, the method for treating hyperlipoidemia comprises administering to a subject in need thereof a therapeutically effective amount of a pharmaceutical composition comprising a therapeutically effective amount of the *Imperata cylindrica* polysaccharide obtained by the present invention .

In one aspect, the present invention also provides an isolated *Imperata cylindrica* polysaccharide for use in the treatment of hyperlipoidemia. In one embodiment, the treatment of hyperlipoidemia refers to regulation of blood lipid metabolism.

The treatment of hyperlipoidemia as described in the present application comprises regulating the blood lipid metabolism, and regulating the blood lipid levels (e.g., lowering the level of lipids in the blood, such as lowering the levels of total cholesterol (TC), triglyceride (TG) and low-density lipoprotein cholesterol (LDL-C) in the blood). Additionally, the *Imperata cylindrica* polysaccharide of the present application can also increase the activities of superoxide dismutase (SOD) and glutathione peroxidase (GSH-px) in a subject (e.g., in serum and liver), and lower the content of malondialdehyde (MDA).

The term "treatment" as used herein generally refers to achieving a desired pharmacological and/or physiological effect. The effect can be prophylactic in terms of complete or partial prevention of a disease or symptoms thereof; and/or can be therapeutic in terms of partial or complete stabilization or curing of a disease and/or side effects due to the disease. The term "treatment" as used herein covers any treatment for a patient's disease, including: (a) preventing a disease or symptom in a patient who is susceptible to the disease or symptom but has not yet been diagnosed with the disease; (b) suppressing the symptom of the disease, i.e., preventing its progression; or (c) alleviating the symptom of the disease, i.e., resulting in regression of the disease or symptom.

Unless otherwise specified, the percentages, proportions, ratios or parts used in the present application are by volume. The volume : weight ratio used in the present application is a volume-to-weight ratio as calculated in milliliter/gram (or liter/kilogram). The concentration used in the present application is a volume concentration.

### BRIEF DESCRIPTION OF THE DRAWINGS

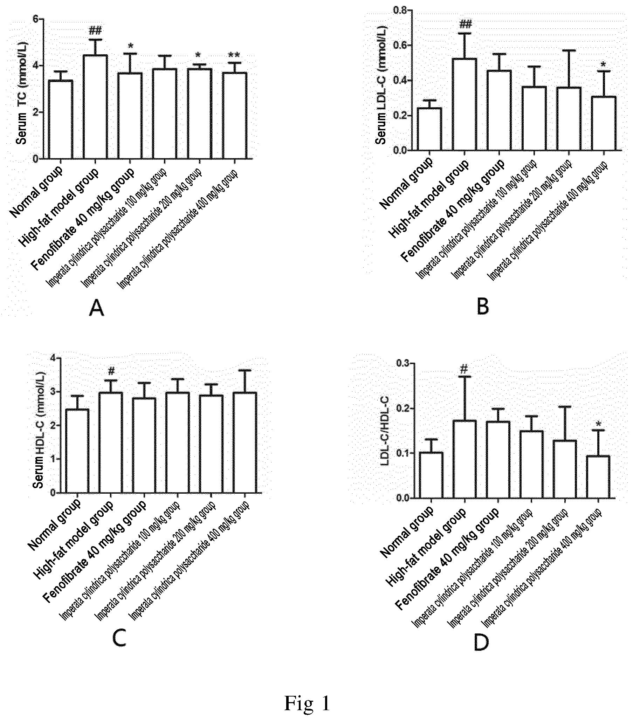

Fig. 1: Effect of *Imperata cylindrica* polysaccharide on blood lipid level in mice with hyperlipoidemia (x̅±S.D, n=10). ^{#}P<0.05 (tested by the LSD method), ^{##}P<0.01 (tested by the LSD method), as compared with the normal control group (Normal group); ^{*}P<0.05 (tested by the LSD method), ^{**}P<0.01 (tested by the LSD method), as compared with the high-fat model group.

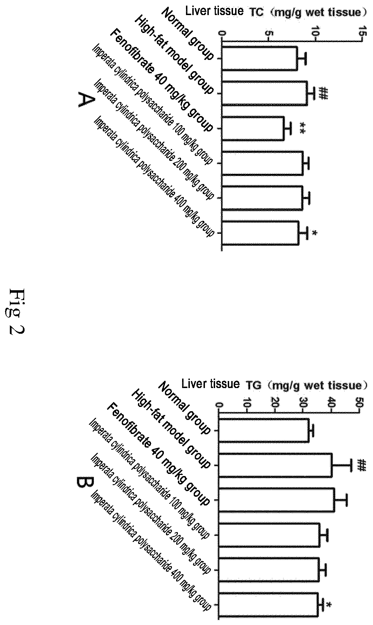

Fig. 2: Effects of *Imperata cylindrica* polysaccharide on liver TC and TG contents in mice with hyperlipoidemia (x̅±S.D, n=10). ^{##}P<0.01 (tested by the LSD method), as compared with the normal control group (Normal group); ^{*}P<0.05 (tested by the LSD method), ^{**}P<0.01 (tested by the LSD method), as compared with the high-fat model group.

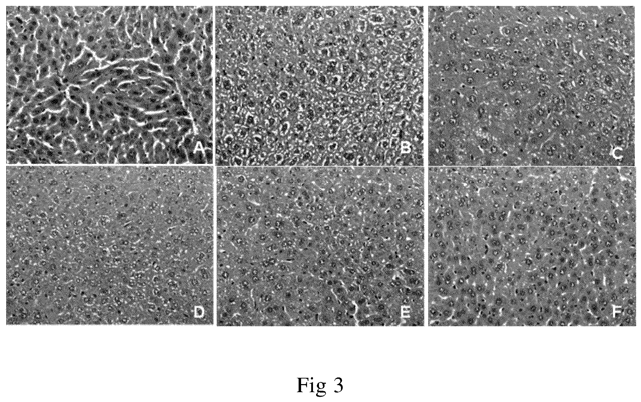

Fig. 3: Effect of *Imperata cylindrica* polysaccharide on liver morphology of mice with hyperlipoidemia. Fig. 3A: Normal control group; Fig. 3B: High-fat model group; Fig. 3C: Lipanthyl 40 mg/kg group; Fig. 3D: *Imperata cylindrica* polysaccharide 100 mg/kg group; Fig. 3E: *Imperata cylindrica* polysaccharide 200 mg/kg group; Fig. 3F: *Imperata cylindrica* polysaccharide 400 mg/kg group.

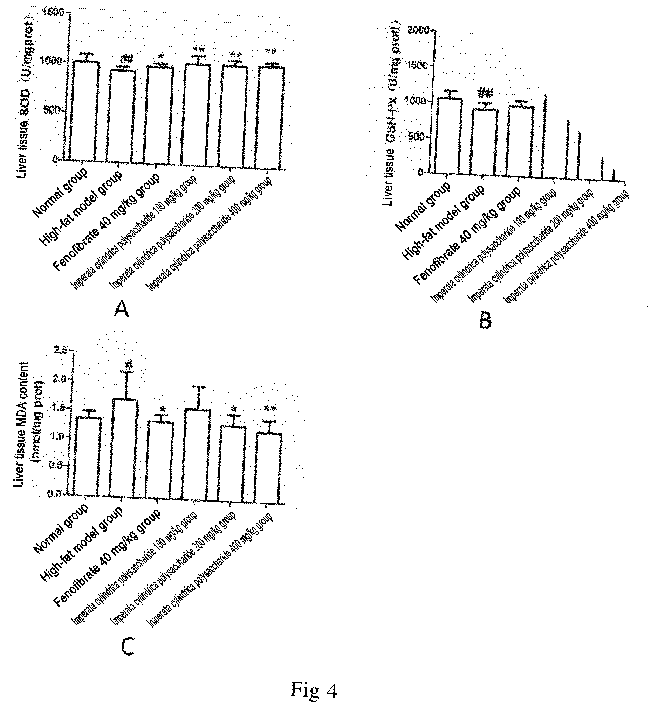

Fig. 4: Effects of *Imperata cylindrica* polysaccharide on liver SOD and GSH-px activities and MDA content in mice with hyperlipoidemia (x̅±S.D, n=10). ^{#}P<0.05 (tested by the LSD method), ^{##}P<0.01 (tested by the LSD method), as compared with the normal control group (Normal group); ^{*}P<0.05 (tested by the LSD method), ^{**}P<0.01 (tested by the LSD method), as compared with the high-fat model group.

### DETAILED DESCRIPTION OF THE INVENTION

The present application will demonstrate the beneficial effects of the present application through Examples below. Those skilled in the art will know that these Examples are illustrative and not limiting. These Examples will not limit the scope of the present application in any way. The experimental operations described in the following Examples are conventional operations, unless otherwise specified; and the reagents and materials are commercially available, unless otherwise specified.

### Main Reagents and Materials

*Imperata cylindrica* decoction pieces were purchased from the traditional Chinese medicinal material market in Bozhou, Anhui, and produced in Anguo. 95% ethanol, hydrochloric acid, sodium hydroxide, Coomassie brilliant blue, sulfuric acid, phenol, barium chloride, trifluoroacetic acid, sodium borohydride, dimethylsulfoxide, etc. were purchased from Sinopharm Chemical Reagent Co., Ltd. The controls such as *L*-arabinose (*L*-Ara), *D*-xylose (*D*-Xyl), *D*-mannose (*D*-Man), *D-*glucose (*D*-Glc), and *D*-galactose (*D*-Gal), and 1-phenyl-3-methyl-5-pyrazolone (PMP) were purchased from Sigma.

### Main Instruments

Model 1260 High Performance Liquid Chromatograph (DAD and RID detectors, Agilent, US); DAWN HELEOS-II 18-Angle Laser Light Scattering Spectrometer (Wayyat, US); Model 7890B Gas Chromatograph-Mass Spectrometer (Agilent, US); Infinite M200 Microplate Reader (Tecan, US).

### Example 1: Preparation of Imperata cylindrica Polysaccharide

(1) 6 L of distilled water was added to 300 g of *Imperata cylindrica* decoction pieces. The *Imperata cylindrica* decoction pieces were extracted with distilled water at 90 °C for 2 h to give an extract. After separation of the extract, the extraction was repeated twice, with 6 L of distilled water for 2 h each time. The resulting extracts were combined and concentrated to 2 L, to give a concentrated extract.
(2) Ethanol was added to the concentrated extract to give a mixture with an ethanol concentration of 20%, and the mixture was centrifuged to give a supernatant.
(3) Ethanol was added to the supernatant to give a mixture with an ethanol concentration of 80%, and the mixture was centrifuged to give a precipitate.
(3') 0.5 L of distilled water was added to the precipitate to dissolve it. Then, ethanol was added again to give a mixture with an ethanol concentration of 80%, and the mixture was centrifuged to give a precipitate.
(4) The resulting precipitate was dried to obtain 12 g of *Imperata cylindrica* polysaccharide, with a yield of 4%.

### Example 2: Preparation of Imperata cylindrica Polysaccharide

(1) 9 L of distilled water was added to 300 g of *Imperata cylindrica* decoction pieces. The *Imperata cylindrica* decoction pieces were extracted with distilled water at 40 °C for 4 h to give an extract. After separation of the extract, the extraction was repeated three times, with 9 L of distilled water for 4 h each time. The resulting extracts were combined and concentrated to 2 L, to give a concentrated extract.
(2) Methanol was added to the concentrated extract to give a mixture with a methanol concentration of 30%, and the mixture was centrifuged to give a supernatant.
(3) Methanol was added to the supernatant to give a mixture with a methanol concentration of 90%, and the mixture was centrifuged to give a precipitate.
(3') 0.5 L of distilled water was added to the precipitate to dissolve it. Then, methanol was added again to give a mixture with a methanol concentration of 90%, and the mixture was centrifuged to give a precipitate. The operation of step (3') was repeated twice.
(4) The resulting precipitate was dried to obtain 9.8 g of *Imperata cylindrica* polysaccharide, with a yield of 3.3%.

### Example 3: Preparation of Imperata cylindrica Polysaccharide

(1) 3 L of distilled water was added to 300 g of *Imperata cylindrica* decoction pieces. The *Imperata cylindrica* decoction pieces were extracted with distilled water at 90 °C for 2 h to give an extract. After separation of the extract, the extraction was repeated 3 times, with 3 L of distilled water for 2 h each time. The resulting extracts were combined and concentrated to 2 L, to give a concentrated extract.
(2) Ethanol was added to the concentrated extract to give a mixture with an ethanol concentration of 15%, and the mixture was centrifuged to give a supernatant.
(3) Ethanol was added to the supernatant to give a mixture with an ethanol concentration of 80%, and the mixture was centrifuged to give a precipitate.
(3') 0.5 L of distilled water was added to the precipitate to dissolve it. Then, ethanol was added again to give a mixture with an ethanol concentration of 80%, and the mixture was centrifuged to give a precipitate. The operation of step (3') was repeated once.
(4) The resulting precipitate was dried to obtain 13.2 g of *Imperata cylindrica* polysaccharide, with a yield of 4.4%.

### Example 4: Preparation of Imperata cylindrica Polysaccharide

(1) 2.4 L of distilled water was added to 300 g of *Imperata cylindrica* decoction pieces. The *Imperata cylindrica* decoction pieces were extracted with distilled water at 100 °C for 1 h to give an extract. After separation of the extract, the extraction was repeated 4 times, with 2.4 L of distilled water for 1 h each time. The resulting extracts were combined and concentrated to 2 L, to give a concentrated extract.
(2) Propanol was added to the concentrated extract to give a mixture with a propanol concentration of 20%, and the mixture was centrifuged to give a supernatant.
(3) Propanol was added to the supernatant to give a mixture with a propanol concentration of 70%, and the mixture was centrifuged to give a precipitate.
(3') 0.5 L of distilled water was added to the precipitate to dissolve it. Then, propanol was added again to give a mixture with a propanol concentration of 70%, and the mixture was centrifuged to give a precipitate.
(4) The resulting precipitate was dried to obtain 10.9 g of *Imperata cylindrica* polysaccharide, with a yield of 3.6%.

### Example 5: Preparation of Imperata cylindrica Polysaccharide

(1) 6 L of distilled water was added to 300 g of *Imperata cylindrica* decoction pieces. The *Imperata cylindrica* decoction pieces were extracted with distilled water at 60 °C for 3 h to give an extract. After separation of the extract, the extraction was repeated twice, with 6 L of distilled water for 3 h each time. The resulting extracts were combined and concentrated to 2 L, to give a concentrated extract.
(2) Propanol was added to the concentrated extract to give a mixture with a propanol concentration of 20%, and the mixture was centrifuged to give a supernatant.
(3) Propanol was added to the supernatant to give a mixture with a propanol concentration of 75%, and the mixture was centrifuged to give a precipitate.
(3') 0.5 L of distilled water was added to the precipitate to dissolve it. Then, propanol was added again to give a mixture with a propanol concentration of 75%, and the mixture was centrifuged to give a precipitate.
(4) The resulting precipitate was dried to obtain 10.2 g of *Imperata cylindrica* polysaccharide, with a yield of 3.4%.

### Example 6: Preparation of Imperata cylindrica Polysaccharide

(1) 6 L of distilled water was added to 300 g of *Imperata cylindrica* decoction pieces. The *Imperata cylindrica* decoction pieces were extracted with distilled water at 80 °C for 2 h to give an extract. After separation of the extract, the extraction was repeated twice, with 6 L of distilled water for 2 h each time. The resulting extracts were combined and concentrated to 2 L, to give a concentrated extract.
(2) Ethanol was added to the concentrated extract to give a mixture with an ethanol concentration of 25%, and the mixture was centrifuged to give a supernatant.
(3) Ethanol was added to the supernatant to give a mixture with an ethanol concentration of 80%, and the mixture was centrifuged to give a precipitate.
(4) The resulting precipitate was dried to obtain 11.8 g of *Imperata cylindrica* polysaccharide, with a yield of 3.9%.

### Example 7: Preparation of Imperata cylindrica Polysaccharide

(1) 9 L of distilled water was added to 300 g of *Imperata cylindrica* decoction pieces. The *Imperata cylindrica* decoction pieces were extracted with distilled water at 70 °C for 2 h to give an extract. After separation of the extract, the extraction was repeated three times, with 9 L of distilled water for 2 h each time. The resulting extracts were combined and concentrated to 2 L, to give a concentrated extract.
(2) Ethanol was added to the concentrated extract to give a mixture with an ethanol concentration of 20%, and the mixture was centrifuged to give a supernatant.
(3) Ethanol was added to the supernatant to give a mixture with an ethanol concentration of 85%, and the mixture was centrifuged to give a precipitate.
(3') 0.5 L of distilled water was added to the precipitate to dissolve it. Then, ethanol was added again to give a mixture with an ethanol concentration of 85%, and the mixture was centrifuged to give a precipitate.
(4) The resulting precipitate was dried to obtain 10.7 g of *Imperata cylindrica* polysaccharide, with a yield of 3.6%.

### Example 8: Preparation of Imperata cylindrica Polysaccharide

(1) 6 L of distilled water was added to 300 g of *Imperata cylindrica* decoction pieces. The *Imperata cylindrica* decoction pieces were extracted with distilled water at 95 °C for 1 h to give an extract. After separation of the extract, the extraction was repeated once, with 6 L of distilled water for 1 h each time. The resulting extracts were combined and concentrated to 2 L, to give a concentrated extract.
(2) Ethanol was added to the concentrated extract to give a mixture with an ethanol concentration of 20%, and the mixture was centrifuged to give a supernatant.
(3) Ethanol was added to the supernatant to give a mixture with an ethanol concentration of 80%, and the mixture was centrifuged to give a precipitate.
(3') 0.5 L of distilled water was added to the precipitate to dissolve it. Then, ethanol was added again to give a mixture with an ethanol concentration of 80%, and the mixture was centrifuged to give a precipitate.
(4) The resulting precipitate was dried to obtain 13.1 g of *Imperata cylindrica* polysaccharide, with a yield of 4.4%.

### Example 9: Structural identification of Imperata cylindrica Polysaccharide

### (1) Determination of the contents of total sugars, uronic acids, proteins and sulfate groups

The content of total sugars of the *Imperata cylindrica* polysaccharides obtained from Examples 1 to 8 were determined by the sulfuric acid-phenol method (see Haixia Wang, Extraction and content determination of Imperata cylindrica polysaccharide, Chinese Journal of Information on Traditional Chinese Medicine, 2010, 17(2): pp. 55-57).

(2) The content of uronic acids of the *Imperata cylindrica* polysaccharides obtained from Examples 1 to 8 were determined by the m-hydroxybiphenyl method (see Lin Gao, Quantitative Determination of Uronic Acid in MCP, Chemical Industry and Engineering, 2005, 22(6): 487-489).

(3) The content of proteins of the *Imperata cylindrica* polysaccharides obtained from Examples 1 to 8 were determined by the Coomassie brilliant blue method (see Jie Zhang, Determination of the basic content of Phellodendron amurense polysaccharides before and after stir-heating with a salt solution and its effect on immune function, Liaoning Journal of Traditional Chinese Medicine, 2017, 44(6): 1263-1267).

(4) The content of sulfate groups of the *Imperata cylindrica* polysaccharides obtained from Examples 1 to 8 were determined by the BaCl₂ turbidimetry (see Qian Chen, Determination of the sulfate content in fucoidan by barium sulfate-turbidimetry, Journal of Pharmaceutical Practice, 2012, 30(2): 118-120).

The measurement results are shown in Table 1 below.

**Table 1**

| Example No. | Content of total sugars (%) | Content of uronic acids | Content of proteins (%) | Content of sulfate groups |
|---|---|---|---|---|
| 1 | 76.53 | n.d. | 2.34 | n.d. |
| 2 | 73.48 | n.d. | 2.76 | n.d. |
| 3 | 77.47 | n.d. | 1.53 | n.d. |
| 4 | 75.35 | n.d. | 2.37 | n.d. |
| 5 | 74.89 | n.d. | 1.44 | n.d. |
| 6 | 76.06 | n.d. | 2.71 | n.d. |
| 7 | 75.92 | n.d. | 1.13 | n.d. |
| 8 | 76.62 | n.d. | 1.53 | n.d. |

### (5) Determination of weight average molecular weights

The weight average molecular weights of the *Imperata cylindrica* polysaccharides obtained from Examples 1 to 8 were determined by the multi-angle laser light scattering method (see Houqiang Ding, Determination of molecular weight and distribution of hyaluronic acid by combination of multi-angle laser light scattering spectrometer and size exclusion chromatography, Food and Drug, 2009, 11(3): 24-26).

### Assay Method

10 mg of the sample to be tested was placed in a 1.5 mL centrifuge tube. Then, 1 mL of deionized water was added to dissolve the sample. The centrifuge tube was centrifuged at 14000 rpm for 10 min to obtain a supernatant. The supernatant was assayed on an Agilent 1260 HPLC chromatograph to determine the weight average molecular weight.

### Chromatographic Conditions:

Chromatographic column: XBridge Protein BEH SEC 200 Å Column (3.5 µm, 7.8×300 mm); column temperature: 25 °C; RID temperature: 35 °C; mobile phase: 0.1 mol/L NaOAc solution; flow rate: 0.5 mL/min; injection volume: 30 µL.

The measurement results are shown in Table 2:

**Table 2**

| Example No. | Weight average molecular weight/Da |
|---|---|
| 1 | 2.2×10⁵ |
| 2 | 7.4×10⁴ |
| 3 | 1.1×10⁵ |
| 4 | 2.4×10⁴ |
| 5 | 8.9×10⁴ |
| 6 | 1.6×10⁵ |
| 7 | 4.3×10⁵ |
| 8 | 2.4×10⁵ |

### (6) Analysis of the monosaccharide composition

2 mg of the *Imperata cylindrica* polysaccharide obtained from Example 1 was dissolved in 1 mL of a 3 mol/L solution of trifluoroacetic acid (TFA) in water in an ampoule, and then the ampoule was sealed. The *Imperata cylindrica* polysaccharide in the ampoule was hydrolyzed at 105 °C for 4 h. After the water in the ampoule was evaporated under reduced pressure to dryness, 2 mL of methanol was added to the ampoule and then evaporated to dryness. The addition of ethanol and evaporation to dryness were repeated twice to remove TFA. Then, 100 µL of water was added to the ampoule to afford a sample of the polysaccharide that was completely hydrolyzed under an acidic condition.

Then, a suitable amount of a monosaccharide control was weighed to prepare a mother solution at a concentration of 1 mg/mL. 10 µL of the mother solution was pipetted and diluted to the constant volume of 100 µL.

Derivatization: To 50 µL of the control solution were added 100 µL of a 0.3 mol/L NaOH solution, 120 µL of a 0.5 mol/L solution of 1-phenyl-3-methyl-5-pyrazolone in methanol sequentially, and mixed well to obtain a mixed solution. The mixed solution was reacted at 70 °C for 60 min. After completion of the reaction, the solution was cooled to room temperature, a suitable amount of 0.3 mol/L HCl was added to adjust the pH to neutral, the solution was extracted with 1 mL of chloroform, and then the organic phase was discarded. 50 µL of the sample of the polysaccharide that was completely hydrolyzed under an acidic condition was subjected to the same derivatization according to the above method.

### Chromatographic Conditions:

Agilent Eclipse XDB-C18 chromatographic column; mobile phase: 0.1 mol/L phosphate buffer (pH=6.7) : acetonitrile (v/v=83:17); column temperature: 25 °C; detection wavelength: 245 nm; flow rate: 1.0 mL/min; injection volume: 10 µL.

The measurement results are shown in Table 3 below:

**Table 3**

| Example No. | Molar ratio (*L*-Ara: *D*-Xyl: *D*-Man: *D*-Glc: *D*-Gal) |
|---|---|
| 1 | 8:3:4:29:45 |
| 2 | 11:14:5:16:43 |
| 3 | 7:3:2:27:50 |
| 4 | 18:3:9:40:55 |
| 5 | 10:5:4:30:47 |
| 6 | 9:2:4:28:45 |
| 7 | 5:4:4:29:46 |
| 8 | 8:2:5:29:46 |

### (7) Methylation Analysis

The *Imperata cylindrica* polysaccharide of Example 1 was methylated by the method as described in the reference (Jinian Fang, Methylation analysis methods of polysaccharides, Foreign Medical Sciences (Section of Pharmacology), 1986, (4): 222-226). The methylated product was depolymerized with 90% formic acid, and fully hydrolyzed with 2 mol/L TFA to afford methylated monosaccharides. Then, the resulting methylated monosaccharides were reduced with NaBH₄ and acetylated with acetic anhydride to generate alditol acetate derivatives of the methylated monosaccharides. Then, the derivatives were subjected to GC-MS analysis.

It could be determined from the results of the methylation analysis that the *Imperata cylindrica* polysaccharides obtained from Examples 1-8 mainly comprised five monosaccharides: *L*-arabinose, *D*-xylose, *D*-mannose, *D*-glucose and *D*-galactose, which were connected by the following monosaccharide form: terminal *L*-arabinose, 1,2,4-*L*-arabinose, terminal *D*-xylose, 1,3,4-*D*-xylose, terminal *D*-mannose, 1,6-*D*-mannose, 1,4-*D*-glucose, 1,6-*D*-glucose, 1,4-*D*-galactose, and 1,4,6-*D-*galactose. The methylation analysis results are shown in Tables 5-12.

**Table 4 Methylation analysis results of the Imperata cylindrica polysaccharide obtained from Example 1**

| Methylated sugar residue | Monosaccharide | Molar ratio |
|---|---|---|
| 2,3,4-Me₃-L-Ara | terminal L-arabinose | 4 |
| 3-Me-L-Ara | 1,2,4-L-arabinose | 4 |
| 2,3,4-Me₃-D-Xyl | terminal D-xylose | 1 |
| 2-Me-D-Xyl | 1,3,4-D-xylose | 2 |
| 2,3,4,6-Me₄-D-Man | terminal D-mannose | 1 |
| 2,3,4-Me₃-D-Man | 1,6-D-mannose | 3 |
| 2,3,6-Me₃-D-Glc | 1,4-D-glucose | 24 |
| 2,3,4-Me₃-D-Glc | 1,6-D-glucose | 5 |
| 2,3,6-Me₃-Gal | 1,4-D-galactose | 20 |
| 2,3-Me₂-D-Gal | 1,4,6-D-galactose | 25 |

**Table 5 Methylation analysis results of the Imperata cylindrica polysaccharide obtained from Example 2**

| Methylated sugar residue | Monosaccharide | Molar ratio |
|---|---|---|
| 2,3,4-Me₃-L-Ara | terminal L-arabinose | 10 |
| 3-Me-L-Ara | 1,2,4-L-arabinose | 1 |
| 2,3,4-Me₃-D-Xyl | terminal D-xylose | 4 |
| 2-Me-D-Xyl | 1,3,4-D-xylose | 10 |
| 2,3,4,6-Me₄-D-Man | terminal D-mannose | 2 |
| 2,3,4-Me₃-D-Man | 1,6-D-mannose | 3 |
| 2,3,6-Me₃-D-Glc | 1,4-D-glucose | 15 |
| 2,3,4-Me₃-D-Glc | 1,6-D-glucose | 1 |
| 2,3,6-Me₃-Gal | 1,4-D-galactose | 13 |
| 2,3-Me₂-D-Gal | 1,4,6-D-galactose | 30 |

**Table 6 Methylation analysis results of the Imperata cylindrica polysaccharide obtained from Example 3**

| Methylated sugar residue | Monosaccharide | Molar ratio |
|---|---|---|
| 2,3,4-Me₃-L-Ara | terminal L-arabinose | 2 |
| 3-Me-L-Ara | 1,2,4-L-arabinose | 5 |
| 2,3,4-Me₃-D-Xyl | terminal D-xylose | 2 |
| 2-Me-D-Xyl | 1,3,4-D-xylose | 1 |
| 2,3,4,6-Me₄-D-Man | terminal D-mannose | 1 |
| 2,3,4-Me₃-D-Man | 1,6-D-mannose | 1 |
| 2,3,6-Me₃-D-Glc | 1,4-D-glucose | 22 |
| 2,3,4-Me₃-D-Glc | 1,6-D-glucose | 5 |
| 2,3,6-Me₃-Gal | 1,4-D-galactose | 15 |
| 2,3-Me₂-D-Gal | 1,4,6-D-galactose | 25 |

**Table 7 Methylation analysis results of the Imperata cylindrica polysaccharide obtained from Example 4**

| Methylated sugar residue | Monosaccharide | Molar ratio |
|---|---|---|
| 2,3,4-Me₃-L-Ara | terminal L-arabinose | 8 |
| 3-Me-L-Ara | 1,2,4-L-arabinose | 10 |
| 2,3,4-Me₃-D-Xyl | terminal D-xylose | 2 |
| 2-Me-D-Xyl | 1,3,4-D-xylose | 1 |
| 2,3,4,6-Me₄-D-Man | terminal D-mannose | 1 |
| 2,3,4-Me₃-D-Man | 1,6-D-mannose | 8 |
| 2,3,6-Me₃-D-Glc | 1,4-D-glucose | 30 |
| 2,3,4-Me₃-D-Glc | 1,6-D-glucose | 10 |
| 2,3,6-Me₃-Gal | 1,4-D-galactose | 25 |
| 2,3-Me₂-D-Gal | 1,4,6-D-galactose | 30 |

**Table 8 Methylation analysis results of the Imperata cylindrica polysaccharide obtained from Example 5**

| Methylated sugar residue | Monosaccharide | Molar ratio |
|---|---|---|
| 2,3,4-Me₃-L-Ara | terminal L-arabinose | 5 |
| 3-Me-L-Ara | 1,2,4-L-arabinose | 5 |
| 2,3,4-Me₃-D-Xyl | terminal D-xylose | 3 |
| 2-Me-D-Xyl | 1,3,4-D-xylose | 2 |
| 2,3,4,6-Me₄-D-Man | terminal D-mannose | 1 |
| 2,3,4-Me₃-D-Man | 1,6-D-mannose | 3 |
| 2,3,6-Me₃-D-Glc | 1,4-D-glucose | 27 |
| 2,3,4-Me₃-D-Glc | 1,6-D-glucose | 3 |
| 2,3,6-Me₃-Gal | 1,4-D-galactose | 19 |
| 2,3-Me₂-D-Gal | 1,4,6-D-galactose | 28 |

**Table 9 Methylation analysis results of the Imperata cylindrica polysaccharide obtained from Example 6**

| Methylated sugar residue | Monosaccharide | Molar ratio |
|---|---|---|
| 2,3,4-Me₃-L-Ara | terminal L-arabinose | 5 |
| 3-Me-L-Ara | 1,2,4-L-arabinose | 4 |
| 2,3,4-Me₃-D-Xyl | terminal D-xylose | 1 |
| 2-Me-D-Xyl | 1,3,4-D-xylose | 1 |
| 2,3,4,6-Me₄-D-Man | terminal D-mannose | 1 |
| 2,3,4-Me₃-D-Man | 1,6-D-mannose | 3 |
| 2,3,6-Me₃-D-Glc | 1,4-D-glucose | 25 |
| 2,3,4-Me₃-D-Glc | 1,6-D-glucose | 3 |
| 2,3,6-Me₃-Gal | 1,4-D-galactose | 20 |
| 2,3-Me₂-D-Gal | 1,4,6-D-galactose | 25 |

**Table 10 Methylation analysis results of the Imperata cylindrica polysaccharide obtained from Example 7**

| Methylated sugar residue | Monosaccharide | Molar ratio |
|---|---|---|
| 2,3,4-Me₃-L-Ara | terminal L-arabinose | 3 |
| 3-Me-L-Ara | 1,2,4-L-arabinose | 2 |
| 2,3,4-Me₃-D-Xyl | terminal D-xylose | 3 |
| 2-Me-D-Xyl | 1,3,4-D-xylose | 1 |
| 2,3,4,6-Me₄-D-Man | terminal D-mannose | 1 |
| 2,3,4-Me₃-D-Man | 1,6-D-mannose | 3 |
| 2,3,6-Me₃-D-Glc | 1,4-D-glucose | 25 |
| 2,3,4-Me₃-D-Glc | 1,6-D-glucose | 4 |
| 2,3,6-Me₃-Gal | 1,4-D-galactose | 16 |
| 2,3-Me₂-D-Gal | 1,4,6-D-galactose | 24 |

**Table 11 Methylation analysis results of the Imperata cylindrica polysaccharide obtained from Example 8**

| Methylated sugar residue | Monosaccharide | Molar ratio |
|---|---|---|
| 2,3,4-Me₃-L-Ara | terminal L-arabinose | 2 |
| 3-Me-L-Ara | 1,2,4-L-arabinose | 2 |
| 2,3,4-Me₃-D-Xyl | terminal D-xylose | 1 |
| 2-Me-D-Xyl | 1,3,4-D-xylose | 1 |
| 2,3,4,6-Me₄-D-Man | terminal D-mannose | 2 |
| 2,3,4-Me₃-D-Man | 1,6-D-mannose | 1 |
| 2,3,6-Me₃-D-Glc | 1,4-D-glucose | 16 |
| 2,3,4-Me₃-D-Glc | 1,6-D-glucose | 1 |
| 2,3,6-Me₃-Gal | 1,4-D-galactose | 15 |
| 2,3-Me₂-D-Gal | 1,4,6-D-galactose | 20 |

### Example 10: Experiment on the effect of Imperata cylindrica polysaccharide in lowering blood lipids

Experimental drug: the *Imperata cylindrica* polysaccharide from Example 1 was administered at doses of 100 mg/kg (low dose), 200 mg/kg (medium dose) and 400 mg/kg (high dose), respectively. Experimental reagents: TC, TG, LDL-C, HDL-C, SOD, GSH-px, MDA and Coomassie brilliant blue protein assay kits were all provided by Nanjing Jiancheng Bioengineering Institute.

Experimental animals: healthy male Kunming mice of clean grade (weighed 18-22 g, provided by Shanghai SLAC Laboratory Animal Co., Ltd.).

Experimental instruments: high-speed freezing centrifuge, produced by Eppendorf, Germany; electronic balance, produced by Mettler-Toledo; multifunctional microplate reader, produced by Berten Instrument Co., Ltd., US.

A hyperlipoidemia model was established with reference to the methods as described in the references (Liyan Sun, Zhenliang Liu, Jinxia Sun, et al., Effect of Imperata cylindrica polysaccharide on hypoxia tolerance in mice, China Journal of Hospital Pharmacy, 2008, 28(2): 96-99; Bin Leng, Intervention of immunoregulation and renal fibrosis by Imperata cylindrica polysaccharide in rats with IgA nephropathy, Dissertations of Guilin Medical University, 2013; Shijing Lv, Qicai Long, Deyuan He, et al., Regulation of lymphocyte proliferation and T cell subpopulation by Imperata cylindrica polysaccharide in patients with hepatitis B, [Conference Paper] 2001 - The Second National Academic Conference on Immunology of Traditional Chinese Medicine). Experimental method: The mice were kept at a temperature of 20±2°C and a humidity of 50±5% under 12 hours of light and 12 hours of darkness for 3 days, during which the mice were allowed to access to food and water *ad arbitrium.* The mice were randomly grouped into 6 groups: a normal control group (Normal group), a high-fat model group, a positive drug Lipanthyl (Fenofibrate, 40 mg/kg) group, a low-dose *Imperata cylindrica* polysaccharide group (ICP 100 mg/ kg), a medium-dose *Imperata cylindrica* polysaccharide group (ICP 200 mg/kg), and a high-dose *Imperata cylindrica* polysaccharide group (ICP 400 mg/kg), 10 animals per group. For each dosing group, different doses of the drugs were administered by oral gavage at 0.2 ml/10 g body weight at 8:00 to 9:00 every day. The normal control group and the high-fat diet group were given an equal volume of distilled water. On day 4 post-dosing, except for the normal control group, the mice in each group were given a high-fat diet (containing 20% lard oil, 10% cholesterol, 0.2% propylthiouracil, 20% propylene glycol, and 20% Tween-80) by oral gavage at 0.2 ml/10 g body weight at 14:00 to 15:00 every day for 3 consecutive weeks. At the end of the experiment, the mice were fasted with free access to water for 8 h and then treated. Blood was collected from the orbit, and serum was obtained from the blood by centrifugation. The serum TC, LDL-C and HDL-C levels were measured and the LDL-C/HDL-C ratio was calculated. A part of the liver tissue of the mice was took, homogenized, and measured for liver TC and TG contents, as well as SOD and GSH-px activities and MDA content. Another part of the liver tissue of the mice was took, fixed with 10% formaldehyde, and examined for morphology.

### Experimental Results:

### (1) Effect of Imperata cylindrica polysaccharides on blood lipid level in mice with hyperlipoidemia

As shown in Fig. 1, the serum TC and LDL-C levels of the mice in the high-fat model group were significantly increased (P<0.01) (tested by the LSD method), and the LDL-C/HDL-C ratio was significantly increased (P<0.05) (tested by the LSD method), as compared to the normal control group. As compared with the high-fat model group, the *Imperata cylindrica* polysaccharide could lower the serum TC and LDL-C levels and the LDL-C/HDL-C ratio. In particular, the middle and high doses of the *Imperata cylindrica* polysaccharide could significantly lower the serum TC content (P<0.05) (tested by the LSD method); and the high dose (400 mg/kg) of the *Imperata cylindrica* polysaccharide could significantly lower the serum TC and LDL-C levels and the LDL-C/HDL-C ratio (P<0.05 or P<0.01) (tested by the LSD method).

### (2) Effect of Imperata cylindrica polysaccharide on liver TC and TG contents in mice with hyperlipoidemia

As shown in Fig. 2, the liver TC and TG contents of the mice in the high-fat model group were significantly increased (P<0.01) (tested by the LSD method) as compared with the normal control group. As compared with the high-fat model group, the *Imperata cylindrica* polysaccharide could lower the liver TC and TG contents. In particular, the high dose of the *Imperata cylindrica* polysaccharide could significantly lower the liver TC and TG contents (P<0.05) (tested by the LSD method).

### (3) Effect of Imperata cylindrica polysaccharide on liver morphology of mice with hyperlipoidemia

As shown in Fig. 3, the mice in the normal control group exhibited complete liver structure and clearly visible hepatic cords, and no obvious lipid vacuole was observed (Fig. 3A). After the mice were given a high-fat diet for 3 weeks, a large number of lipid vacuoles were observed in the liver of the mice in the high-fat model group (Fig. 3B). The *Imperata cylindrica* polysaccharide could significantly improve lipid vacuoles in the liver; and the high dose of the *Imperata cylindrica* polysaccharide had a better effect (Figs. 3D, 3E, and 3F).

### (4) Effect of Imperata cylindrica polysaccharide on liver SOD and GSH-px activities and MDA content in mice with hyperlipoidemia

SOD and GSH-px are antioxidant enzymes in the liver, which can reduce the amount of active oxygen species and alleviate the damage of lipid peroxides to liver cells. As shown in Fig. 4, the SOD and GSH-px activities in the liver of the mice in the high-fat model group were both significantly reduced (P<0.01) (tested by the LSD method), and the MDA content was significantly increased (P<0.05) (tested by the LSD method), as compared with the normal control. The *Imperata cylindrica* polysaccharide could increase the liver SOD and GSH-px activities in the mice (P<0.05 or P<0.01) (tested by LSD method) and reduce the content of MDA. In particular, the low dose, middle dose and high dose of the *Imperata cylindrica* polysaccharide could significantly increase the liver SOD and GSH-px activities in the mice (P<0.05 or P<0.01) (tested by the LSD method), and the middle dose and high dose of the *Imperata cylindrica* polysaccharide could significantly reduce the content of MDA (P<0.05 or P<0.01) (tested by the LSD method).

Experimental conclusion: In mice with hyperlipoidemia induced by a high-fat diet, the *Imperata cylindrica* polysaccharide can significantly lower the serum TC and LDL-C levels and the LDL-C/HDL-C ratio; meanwhile, it can also lower the liver TC and TG contents and significantly reduce lipid vacuoles in the liver.

## Claims

1. An isolated *Imperata cylindrica* polysaccharide, comprising *L*-arabinose, *D*-xylose, *D-*mannose, *D*-glucose and *D*-galactose, wherein the molar ratio of the *L*-arabinose: *D*-xylose: *D-*mannose: *D*-glucose: *D*-galactose is 1-20 : 1-15 : 1-15 : 15-40 : 25-60, preferably 5-10 : 1-5 : 1-5 : 25-30 : 45-55.

2. The isolated *Imperata cylindrica* polysaccharide of claim 1, wherein:
the *L*-arabinose includes terminal *L*-arabinose and/or 1,2,4-linked *L*-arabinose;
the *D*-xylose includes terminal *D*-xylose and/or 1,3,4-linked *D*-xylose;
the *D*-mannose includes terminal *D*-mannose and/or 1,6-linked *D*-mannose;
the *D*-glucose includes 1,4-linked *D*-glucose and/or 1,6-linked *D*-glucose; or
the *D*-galactose includes 1,4-linked *D*-galactose and/or 1,4,6-linked *D*-galactose.

3. The isolated *Imperata cylindrica* polysaccharide of claim 2, wherein the molar ratio of the terminal *L*-arabinose: 1,2,4-linked *L*-arabinose: terminal *D*-xylose: 1,3,4-linked *D*-xylose: terminal *D*-mannose: 1,6-linked *D*-mannose: 1,4-linked *D*-glucose: 1,6-linked *D*-glucose: 1,4-linked *D-*galactose: 1,4,6-linked *D*-galactose is 1-10 : 1-10 : 1-5 : 1-10 : 1-5 : 1-10 : 15-30 : 1-10 : 10-25 : 15-30, preferably 1-5 : 1-5 : 1-3 : 1-5 : 1-3 : 1-5 : 20-25 : 1-5 : 15-20 : 20-25.

4. The isolated *Imperata cylindrica* polysaccharide of claim 3, wherein the isolated *Imperata cylindrica* polysaccharide has a molecular weight ranging from 1×10⁴ to 5×10⁵ Da, preferably 1×10⁵ to 3×10⁵ Da.

5. A method for preparing the isolated *Imperata cylindrica* polysaccharide of any one of claims 1-4, wherein the method comprises the steps of:
(1) extracting *Imperata cylindrica* with water one or more times to give an aqueous extract of *Imperata cylindrica,* optionally concentrating the aqueous extract of *Imperata cylindrica;*
(2) adding an organic solvent to the optionally concentrated aqueous extract of *Imperata cylindrica* to give an mixture containing the organic solvent at a concentration of 15-30%, preferably 17-28%, and more preferably 20-25%, centrifuging the mixture to give a supernatant;
(3) adding an organic solvent to the supernatant to give a mixture containing the organic solvent at a concentration of 70-90%, preferably 75-85%, and more preferably 80-85%, centrifuging the mixture to give a precipitate; and
(4) drying the precipitate to obtain the isolated *Imperata cylindrica* polysaccharide.

6. The method of claim 5, wherein the volume : weight ratio of water to the *Imperata cylindrica* in step (1) is 8:1 to 30:1, preferably 20:1 to 30:1.

7. The method of any one of claims 5-6, wherein the extraction temperature in step (1) is 40-100 °C, preferably 60-100 °C, and most preferably 90-95 °C.

8. The method of any one of claims 5-6, wherein the extraction time in step (1) is 1-4 hours, preferably 1-2 hours.

9. The method of any one of claims 5-6, wherein in step (1), the *Imperata cylindrica* is extracted with water one or more times, preferably 1-4 times, and most preferably 2-3 times.

10. The method of claim 5, wherein the method further comprises step (3') between steps (3) and (4): dissolving the precipitate obtained from step (3) with water to give an aqueous solution, adding an organic solvent to the aqueous solution to give a mixture containing the organic solvent at a concentration of 70-90%, preferably 75-85%, and more preferably 80-85%, and centrifuging the mixture to give a precipitate; wherein step (3') can be repeated one or more times, preferably 1, 2 or 3 times.

11. The method of claim 5 or 10, wherein the organic solvent in step (2) and/or (3) and/or (3') is selected from methanol, ethanol, propanol, acetone, or a mixture thereof, preferably ethanol.

12. The method of any one of claims 5-6, wherein the *Imperata cylindrica* in step (1) is a decoction piece of *Imperata cylindrica.*

13. Use of the isolated *Imperata cylindrica* polysaccharide of any one of claims 1-4 in the manufacture of a medicament for treating hyperlipoidemia.

14. A pharmaceutical composition comprising the isolated *Imperata cylindrica* polysaccharide of any one of claims 1-4, and a pharmaceutically acceptable carrier.

15. Use of the pharmaceutical composition of claim 14 in the manufacture of a medicament for treating hyperlipoidemia.

16. The isolated *Imperata cylindrica* polysaccharide of any one of claims 1-4 for use in the treatment of hyperlipoidemia.

17. A method for treating hyperlipoidemia, comprising administering to a subject in need thereof a therapeutically effective amount of the isolated *Imperata cylindrica* polysaccharide of any one of claims 1-4.
